# EUROPEAN PATENT APPLICATION

(11) **EP 1 593 965 A2**
(43) Date of publication of application: **09.11.2005**
(21) Application number: 04026037.4
(22) Date of filing: 03.11.2004
(51) Int. Cl.: G01N 33/49

(54) **Method and apparatus for storing and transporting density gradient solutions**

(30) Priority: 04.11.2003 US 700933
(71) Applicant: Teledyne Isco, Inc., Lincoln, Nebraska 68504 (US)
(72) Inventor: Davison, Dale A., Greenwood, NE 68366 (US); Liescheski, Phillip B., Lincoln, NE 68506 (US)
(74) Representative: UEXKÜLL & STOLBERG

(57) **Abstract**

To perform density gradient separation, density gradient columns are prepared at a central location and frozen. They are then taken to other locations while frozen and heated to return the mobility of the density gradient solutions. In this manner, the density gradient solutions can be preserved during travel and for a long period of time, thus removing the need for forming the density gradient solutions at the sites they are to be used and at the time of use and avoiding the need for the purchase of specialized gradient forming equipment in some facilities that use solvent gradient density separations.

## Description

### BACKGROUND OF THE INVENTION

This invention relates to density gradient tubes, methods of forming, using and transporting density gradient tubes and apparatus for forming, using and transporting gradient tubes from place to place.

Density gradient solution tubes have long been used to separate different density components of mixtures. Apparatuses for automatically forming density gradient solutions such as density gradient solutions of sucrose or cesium chloride solutions and apparatuses for detecting and collecting fractionated components of a mixture separated in a density gradient tube such as by density gradient centrifugation are known. For example, one apparatus for automatically forming a density gradient solution is disclosed in United States Patent 4,753,892 and an apparatus for detecting and collecting fractionated components of the sample mixture after density gradient centrifugation is disclosed in United States Patent 3,151,639.

In the prior art use of density gradient solutions separation, the density gradient solutions are formed in density gradient tubes at or near the location of use and used, such as for fractionation by density gradient centrifugation, very soon after formation of the gradient in the tube and before the gradient degrades significantly. This prior art method has several disadvantages, such as for example: (1) it requires expensive equipment on site to form gradients as needed; and (2) it requires considerable time of skilled and well paid scientists and technicians to prepare the gradients.

It is known from Baxter-Gabbard, K. L. FEBS LETTERS, v. 20, n.1. January 1972, to cycle sucrose solutions through freeze-thaw cycles. This process forms sucrose gradients. This process was described as a simple way of providing a large number of gradients to be used in a short time at one laboratory and there is no suggestion of supplying remote laboratories from a central laboratory.

It is an object of the invention to provide a novel method of providing density gradient solutions.

To accomplish this object, density gradient separations are performed by at least partly forming and immobilizing at least one part of a density gradient solution and re-mobilizing the density gradient solution before using the density gradient solution for a separation process. The density gradient solution may be subjected to conditions that under some circumstances would degrade the density gradient solution if it were not immobilized.

Advantageously, a plurality of immobilized density gradient solutions are prepared at a central station and transported while immobilized to at least one remote station. Different ones of the immobilized gradient solutions are shipped to correspondingly different remote stations. In one embodiment, layers of different density fluids are formed of different density fluids and immobilized. The fluids of different densities are mixed in a series of different proportions to form a series of different density layers. In another embodiment, the different density fluids are brought close to the freezing point and layered into a container sufficiently cold to immobilize the layers as they are inserted into the container. In one alternative process, a density gradient solution may be formed with a density gradient former and frozen before defusion degrades it.

In another embodiment, at least portions of different density material may be frozen and packaged in shipping packages to be shipped to other locations for assembly into density gradient solution tubes. More specifically, immobilized fluid units of different densities having a size appropriate for inserting in a density gradient tube may be gathered and inserted with an immobilized unit of highest density inserted first followed with successively lower density units, wherein an immobilized density gradient solution tube is formed. The units may then be re-mobilized in the density gradient tube at a remote location and the re-mobilized units allowed to defuse together after re-mobilization to form the density gradient solution. The units are re-mobilized by thawing.

More specifically, at least one part of a density gradient solution is formed and immobilized by drawing a first fluid from a high density fluid source; drawing a second fluid from a lower density fluid source; mixing the fluids and layering them into a liquid density gradient tube. Tthis process is repeated while changing from a large amount of the highest density fluid to lower and lower proportions of the high density fluid whereby layers of different proportions of fluid are applied to the liquid density gradient tube. The fluids in the density gradient tube are immobilized before the layers diffuse together. The fluids may be immobilized by freezing the fluids or in the alternative each of the layers may be precooled and applied under circumstances that cause immobilization.

The apparatus for performing density gradient separations described above may comprise a gradient former, apparatus for storing gradient tubes with immobilized density gradients in them and apparatus for immobilizing gradients formed by said gradient former in gradient tubes. The gradient former and the apparatus for immobilizing gradients formed by said gradient former in gradient tubes may be located in a central station and sent to a plurality of remote stations, with each of the remote stations including at least one storage apparatus for storing said gradient tubes, apparatus for re-mobilizing said density gradient solution prior to performing density gradient separations and apparatus for performing density gradient separations. The gradient former includes apparatus for mixing at least first and second fluids having different densities and control apparatus for controlling the apparatus for mixing to prepare a predetermined density gradient.

The gradient former may include a first pump, a second pump, a first source of fluid having a predetermined density, a second source of fluid having a second predetermined density and a mixer. The first pump communicates with the first source of fluid to pump fluid into said mixer and the second pump communicates with the second source of fluid to pump a second fluid into the mixer. A control unit causes the pumps to form a plurality of fluids of different densities and the gradient former includes an apparatus for applying a plurality of different density layers to at least one density gradient tube, an apparatus for immobilizing the fluids in said density gradient tube and an apparatus for mixing said different layers in said density gradient tubes to form a continuous gradient in said tube. The apparatus for immobilizing includes apparatus freezing. An insulated package is used to ship gradient tubes with immobilized density gradients in them or individual units of different densities to be used in assembling a gradient in a gradient tube at the remote station.

As can be understood from the above description, the technique of this invention has several advantages, such as for example: (1) it reduces the amount of time that skilled personnel must spend in forming density gradient solutions; (2) it reduces the cost of density gradient separations; and (3) it permits greater flexibility in the formation and use of density gradient solutions.

### BRIEF DESCRIPTION OF THE DRAWINGS

The above noted other features of the invention will be better understood from the following detailed description when considered with reference to the drawings, in which:
FIG. 1 is a block diagram of a system for performing gradient density separations in accordance with an embodiment of the invention;
FIG. 2 is a block diagram of a process for forming and immobilizing a density gradient solution in accordance with one embodiment of the invention;
FIG. 3 is a block diagram of another embodiment for forming and immobilizing a density gradient solution;
FIG. 4 is a block diagram of an embodiment for performing the process of FIG. 1;
FIG. 5 is a block diagram of a system for forming a gradient in accordance with an embodiment of the invention; and
FIG. 6 is a simplified diagram of an apparatus for re-mobilizing an immobilized gradient in accordance with an embodiment of the invention.

### DETAILED DESCRIPTION

Briefly, in practicing this invention, density gradient solutions are formed or partly formed or parts of density gradient solutions are formed and immobilized, after which, they may be moved to a new location and utilized or may be left standing where formed for a reasonably long period of time before actual use. In one embodiment, the gradients are formed and immobilized such as by freezing to preserve the gradient until it is ready for use at that location or to permit it to be moved to a new location for use without disrupting the density gradient solution. The density gradient solution may be partly formed before freezing such as by forming layers of different density, which upon melting will merge to form a smooth gradient or the smooth gradient may first be formed and then frozen. Moreover, portions of a gradient may be frozen and then stacked one on the other in a gradient tube and permitted to melt and merge into a smooth gradient at a new location. All of these techniques provide a practical method whereby gradients or parts of gradients may be formed at a central location and then shipped.

This procedure of forming, immobilizing, storing or moving the immobilized gradients and re-mobilizing the density gradient solutions for use provides flexibility to the scientist in quickly forming gradients with shorter amounts of time. For example, entire density gradient solutions in centrifugation tubes may be stored in a freezer or portions of gradients may be stored in a freezer for assembly and use later or partly formed density gradient solutions may be formed and stored in a freezer for completion later. A laboratory may store several of the density gradient solutions that it commonly uses or may order them for quick delivery as needed. The scientist may order gradients from a catalog of common standard sizes or may have them custom made quickly and efficiently at the central location for shipment to the scientist's laboratory.

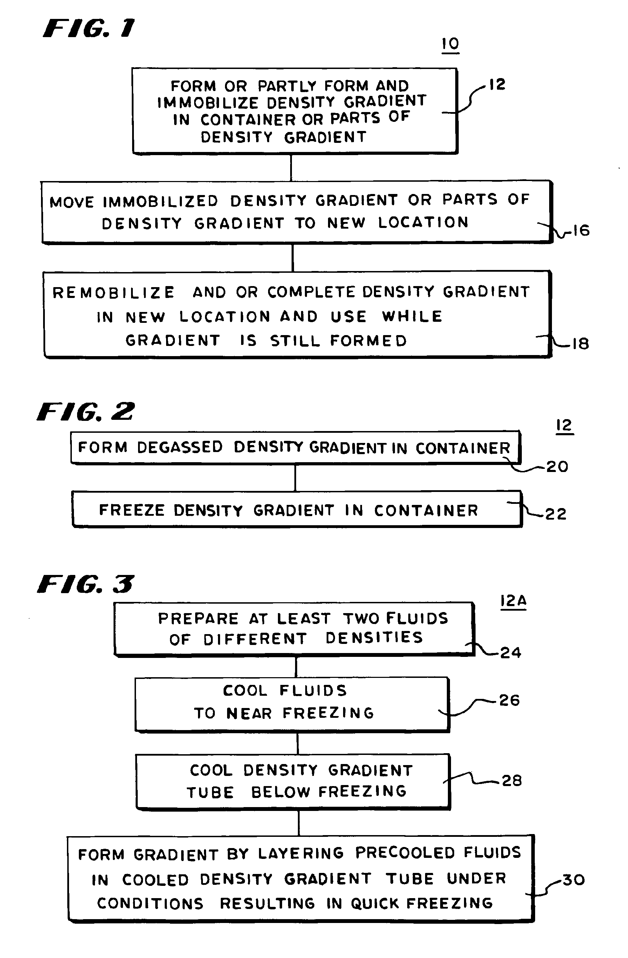

In FIG. 1, there is shown a block diagram of a process 10 for using density gradient solutions including the step 12 of forming or partly forming and immobilizing density gradient solutions or parts of density gradient solutions, the step 16 of moving the density gradient solution or parts of a density gradient solution to a new location and the step 18 of re-mobilizing the density gradient solutions for use in separating components of a mixture by density. The density gradient solutions may be formed completely prior to immobilization such as by using any of the commercially available automatic density gradient formers. One such gradient density is formerly described in U.S. Patent 4,753,892 to Coombs issued June 28, 1988. Generally the solution must be degassed before immobilization to avoid excessive disturbance of the density gradient solution by bubbles during re-mobilization. Degassing can be accomplished using known degassing techniques such as that disclosed in United States Patent 3,751,879 to Allington, issued August 14, 1973.

The system of FIG. 1 includes several variations of the step 12 of forming or partly forming and immobilizing density gradient solutions or parts of density gradient solutions. The density gradient solution may be, for example: (1) completely formed with the pre-planned final length of the column and with the pre-planned rate or rates of change of concentration along the length of the column and then immobilized before storing or moving; or (2) may be formed in sections each of which is less than the pre-planned final length but with the final rate of change of concentration and immobilized for assembly of sections into a density gradient solution with the pre-planned length and gradient after being moved or stored for a time but before re-mobilization; or (3) formed with one or more sections having shorter than the pre-planned final length and with a rate of change of concentrations different from that pre-planned length for assembly after moving or storing the section but before re-mobilizing and for forming the planned rate of change of concentration after re-mobilizing; or (4) with the pre-planned final length of column but not having the pre-planned final rate of change of concentration along its length for correction ofthe concentration after re-mobilization. Still other variations may be possible. In this specification, "rate of change" as applied to concentration of density of solutions means every change including several rates of change or several different gradients or abrupt changes. Similarly, gradient may mean any change in density or concentration.

The immobilization of the density gradient solution or part of density gradient solution may be accomplished in several different ways. In the preferred embodiment, the immobilization is done by freezing, but other immobilization techniques or combinations of immobilization techniques can be used as described below. In some embodiments, the gradient may be immobilized as it is formed. It can be immobilized as it is formed by depositing layers of different densities one on the other in a highly porous material or by applying layers of cold density gradient solutions of different concentrations one over the other in a cold gradient density tube so they freeze as applied. It is even possible to permit separation while the gradient is in its immobilized form in a porous matrix. Moreover, matrix may be formed of material that can be chemically broken down or removed by radiation or by a temperature change to re-mobilize an already formed density gradient solution. In some embodiments a gel may be utilized or the combination of a viscous material and cold temperatures or large porous electrically resistive sections containing a density gradient solution immobilized within it because of the matrix or because of a combination of the matrix and cold temperatures. The matrix and density gradient solution in the matrix that is frozen or viscous because of cold temperature can be re-mobilized by passing an electric current through the matrix to evenly heat it. In another embodiment, the gradient may be formed and then chemically converted into a gel right after forming, moved to a new location and then chemically returned to a more mobile state.

Immobilization in this specification means that the gradient layers are not mixed to an extent to prevent a useful separation by density of the components of a sample even when subjected to stress such as by mild jolting during transportation or shaking or the like. In some cases, this may be referred to as a hard freeze, but in other circumstances where there is going to be minor influences that would cause mixing, it can only mean putting the material in a highly viscous state for the time needed and to withstand the amount of vibrations or shaking or disruptions that may occur. The density gradient solution is re-mobilized when impediments to the separation of components of a sample by density in it are removed to a sufficient extent to permit practical separation. When the density gradient solutions or partly formed density gradient solutions or parts of density gradient solutions have been immobilized, they may be maintained in that state until ready for use. Advantageously, they can be shipped even through long distances and quickly put into use without the expenditure oftime or the use of special gradient forming equipment at a laboratory as shown in step 16.

The step 16 of moving density gradient solutions or parts of density gradient solutions to a new location can also be performed in different modes. For example, density gradient solutions or parts of density gradient solutions can be prepared and immobilized in a facility and stored until it is pre-planned to use them. They can be then removed from storage, re-mobilized in the facility and used as shown in step 18. Density gradient solutions can also be partly formed and/or parts of density gradient solutions can be formed or partly formed and immobilized in a central facility and shipped to one or more remote facilities or locations where they can be re-mobilized for use or assembled, re-mobilized and used or assembled, re-mobilized and completed such as by smoothing the gradient with diffusion and used.

In partly forming density gradient solutions for immobilization and storage or shipment partly formed for completion later, layers of different densities are formed one on top of the other with relatively sharp transitions between the layers and immobilized in that form. With this embodiment, the density gradient solution is re-mobilized and then caused or permitted to form a continuous gradient by the gradual merger of one layer into the other such as for example by diffusion. Also, complete gradient density tubes need not be formed before immobilizing but smaller sections such as for example a series of different density sections can be immobilized and stored or shipped to another location in the same or different packages. After being stored or shipped, the sections can be assembled at the site for use and re-mobilized in accordance with the need of the researcher at the time for performing the separation. This permits a research laboratory to store a large number of immobilized different density sections for assembly at any time relatively easily. For example the researcher can select a section of the highest density pre-planned and than assemble successively lower density sections in order above the highest density section. The stack of sections can then be immobilized and permitted or caused to combine into a continuous gradient. The step 18 will generally be performed just before use of the density gradient solution in a separation process. In some cases, the immobilized solutions will be layered and will have to be permitted to stand or a gradient forming machine used to finish forming the gradient. Because thermal discontinuities during melting of frozen gradient density solutions can result in convection of the liquid that may disrupt the gradient in time, the warming of the frozen gradient or gradient parts should be done as uniformly as possible throughout the cross-section of the gradient tube. For this reason, the warming may be done in a water bath or other specialized equipment.

In FIG. 2, there is shown a block diagram of one embodiment 12 of the process of forming or partly forming an immobilized gradient including the step 20 of forming a degassed density gradient solution in a container. While it is not necessary in some embodiments to form the complete density gradient solution in a container such as a gradient tube, the embodiment 12 has the advantages of permitting quick use at a distant site by the researcher. In embodiments where only part of a gradient is formed and then shipped for insertion into an empty gradient tube in sections, further steps are needed to complete the density gradient solution in the proper separation tube for use whereas in the embodiment of FIG. 1 it is only necessary to re-mobilize the density gradient solution prior to use, which in a preferred embodiment may be done simply by increasing the temperature of a frozen gradient until it is sufficiently mobile. Moreover, at the central location where the density gradient solution is prepared, the complete gradient may be quickly formed using automatic equipment as described above. Once the gradient is formed but before it has time to be altered significantly by dispersion, it can be frozen hard as shown in step 22 or immobilized by some other technique.

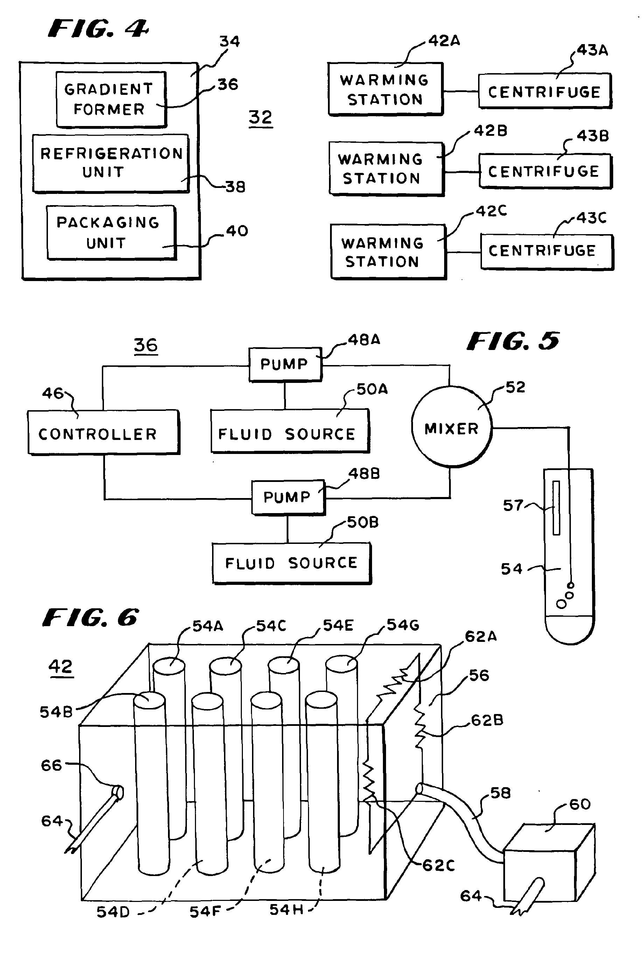

In FIG. 3, there is shown another embodiment 12A of the step of forming and immobilizing a density gradient solution including the step 24 of preparing at least two fluids of different densities such as two sucrose solutions of different concentrations of sucrose, the step 26 of cooling both of the solutions to near freezing, the step 28 of cooling the density gradient tube to freezing or below freezing and the step 30 of forming a partially complete immobilized gradient by layering the precooled fluids in a cooled density gradient tube under conditions resulting in quick freezing so that the result is an immobilized density gradient tube with layers of different density which will by dispersion form a gradient upon warming.

In FIG. 4, there is shown a block diagram 32 of an apparatus for utilizing density gradient solutions having a forming site or central station 34 and one or more warming stations shown at 42A, 42B, and 42C, each with a corresponding one of the centrifuges 43A-43C. Of course only one warming station may be utilized but generally there will be one central station and a large number of warming stations in different parts of the country or different parts of the world to which gradients are shipped for use at the warming station. The central station 34 typically includes a gradient former 36 for automatically forming gradients, a refrigeration unit or other immobilization equipment 38 and a packaging unit 40 for packaging together immobilized containers such as gradient tubes packed in dry ice boxes in the manner other frozen goods such as frozen foods are shipped. These may then be shipped by the normal commercial approaches for shipping frozen goods such as frozen foods such as by commercial courier or specialized trucks or aircraft.

In FIG. 5, there is shown a system 36 for automatically forming gradients having a controller 46, first and second pumps 48A and 48B, first and second fluid sources 50A and 50B, a mixer 52 and a density gradient tube 54. The pumps 48A and 48B are controlled by the controller 46 to cause the pump 48A to pump a fixed amount of high density solution from the fluid source 50A and for pump 48B to pump a lower density solution from the fluid source 50B into the mixer 52. The amounts of the high density and low density solutions are varied by the controller 46 so that the mixer 52 applies different density layers to the tube 54 under the control of the controller 46 for immobilization and shipping. A strip 57 of a substance that changes color or melts and runs upon heating can be located on a tube after the gradient is frozen or located elsewhere with the tubes so that the recipient can be assured the frozen density gradients have not melted in transit and thus have not lost their stable state. Where the size of the shipment justifies the cost, an inexpensive temperature indicator or recorder can be included in the package such as the DS1615 temperature recorder sold by Maxim Integrated Products, 120 San Gabriel Dr., Sunnyvale, CA 94086.

In FIG. 6, there is shown an embodiment of a warmer 42 comprising a warming block 56 which may be of metal, a plurality of openings 54A - 54H in the warming block 56 to receive density gradient tubes 54 (shown in FIG. 5) having frozen density gradient solutions in them. The block 56 has an electrical resistant warming cable 58 powered by a power supply 60 connected electrically to the resistance wires 62A-62C. The power supply 60 is used to apply electrical current to the resistance wires 62A-62C at a rate to gradually warm the metal block 56 so as to apply uniform heat to the density gradient tubes 54. The block 56 is maintained at a preset temperature by a feedback system having a temperature sensor 66 connected to the power supply 60 by a conductor 64. This process is intended to avoid thermal gradients within the density gradient tubes 54 which may cause one section to become immobilized before another. The immobilization of one portion of the frozen density gradient solution before another could cause mixing to reduce the uniformity of the density gradient solution. Of course other warming techniques can be utilized such as a water bath or the like.

While the preferred embodiment utilizes a single immobilization technique such as freezing, multiple techniques can be utilized for the same density gradient solution or density gradient solution part. For example, a chemical immobilization technique may be utilized together with a temperature technique with the two combining to form the pre-planned viscosity. The lowering of temperature may not only accomplish freezing but may be utilized to trigger a reaction forming a gel in which case the cycle may be to warm the reaction mixture and then cool the reaction mixture with the re-mobilization being, as an example, heating to a higher temperature or the use of still another chemical or the use of radiation or the like.

From the above description, it can be understood that, there are several advantages to the system and apparatus for utilizing density gradient solutions, such as for example: (1) under some circumstances it may avoid the necessity of laboratories buying expensive gradient forming equipment; (2) it reduces the amount of time that researchers need to spend doing the relatively menial task of forming density gradient solutions; and (3) it permits the formation of specialized density gradient solutions such as density gradient solutions with different variations and in some circumstances perhaps even different gradient materials such as may be formed in parts and frozen at a site and then withdrawn from stock in different combinations at remote laboratories.

While a preferred embodiment has been described with some particularity, it should be understood that, within the scope of the appended claims, the invention may be practiced other than as specifically described.

## Claims

**1.** A method of performing density gradient separations comprising the steps of: at least partly forming and immobilizing at least one part of a density gradient solution; re-mobilizing the density gradient solution before using the density gradient solution for a separation process; and utilizing the density gradient solution in a separation of process **characterized in that** the density gradient solution may be subjected to conditions that under some circumstances would degrade the density gradient solution if it were not immobilized.

**2.** A method in accordance with claim 1 **characterized in that** a plurality of immobilized density gradient solutions are prepared at a central station and transported while immobilized to at least one remote station.

**3.** A method in accordance with either claim 1 or claim 2 **characterized in that** the different ones of the immobilized gradient solutions are shipped to correspondingly different remote stations.

**4.** A method in accordance with any of claims 1 through 3 **characterized in that** the step of at least partly forming the density gradient solution includes the step of forming layers of different density fluids and immobilizing the layers of different density fluids.

**5.** A method in accordance with claim 4 **characterized in that** step of forming layers of different density fluids includes the step of mixing fluids of different densities in a series of different proportions to form a series of different density layers.

**6.** A method in accordance with any of claims 1-5 **characterized in that** the different density fluids are brought close to the freezing point and layered into a container sufficiently cold to immobilize the layers as they are inserted into the container.

**7.** A method in accordance with any of claims 1-6 **characterized in that** the step of at least partly forming at least one part of a density gradient solution includes the step of forming a density gradient solution with a density gradient former and freezing the density gradient solution before defusion degrades it.

**8.** A method in accordance with any of claims 1-7 **characterized in that** the step of at least partly forming and immobilizing at least one part of a density gradient solution includes the step of freezing at least portions of different density material; said method further including the steps of : packaging the different density portions; and shipping packages of different density portions to other locations for assembly into density gradient solution (54A-54H) (54A-54H).

**9.** A method in accordance with claim 1 **characterized in that** the steps of at least partly forming and immobilizing at least one part of a density gradient solution includes the steps of: gathering immobilized fluid units of different densities having a size appropriate for inserting in a density gradient tube (54A-54H); inserting a highest density immobilized unit into the tube (54A-54H); and following it with successively lower density units, wherein an immobilized density gradient solution tube (54A-54H) is formed.

**10.** A method in accordance with claim 9 **characterized by** the steps of: re-mobilizing the units in the density gradient tube (54A-54H); and permitting the re-mobilized units to defuse together after re-mobilization to form said density gradient solution.

**11.** A method in accordance with claim 10 **characterized in that** the step of re-mobilizing a unit comprises the step of thawing a unit.

**12.** A method in accordance with claim 1 **characterized in that** the steps of at least partly forming and immobilizing at least one part of a density gradient solution includes the steps of: drawing fluid from a high density fluid source; drawing a second fluid from a lower density fluid source; mixing the fluids and layering them into a liquid density gradient tube (54A-54H); repeating the process while changing from a large amount of the highest density fluid to lower and lower proportions whereby layers of different proportions of fluid are applied to the liquid density gradient tube (54A-54H); and immobilizing the fluids in the density gradient tube (54A-54H) before the layers diffuse together.

**13.** A method in accordance with claim 12 **characterized in that** the step of immobilizing the fluids comprises the step of freezing the fluids.

**14.** A method in accordance with claim 12 **characterized in that** each of the layers are precooled and applied under circumstances that cause immobilization.

**15.** Apparatus for performing density gradient separations comprising a gradient former and an apparatus for storing density gradient tubes (54A-54H) with immobilized density gradients in them **characterized by** apparatus for immobilizing gradients formed by said gradient former in gradient tubes (54A-54H); said gradient former and apparatus for immobilizing gradients formed by said gradient former in gradient tubes (54A-54H) being located in a central station; a plurality of remote stations; each of said remote stations including at least one apparatus for storing said gradient tubes (54A-54H) and an apparatus for re-mobilizing said density gradient solution prior to performing density gradient separations and an apparatus for performing density gradient separations.

**16.** Apparatus in accordance with claim 15 **characterized in that** said gradient former comprises: an apparatus for mixing at least first and second fluids; said first and second fluids having different densities; a control apparatus for controlling said apparatus for mixing to prepare a predetermined density gradient.

**17.** Apparatus for forming gradients comprising: a first pump; a second pump; a first source of fluid having a predetermined density; a second source of fluid having a second predetermined density; a mixer; said first pump communicating with said first source of fluid to pump fluid into said mixer; said second pump communicating with said second source of fluid to pump a second fluid into said mixer; a control unit for controller; **characterized in that** the control unit causes said pumps to form a plurality of fluids of different densities said gradient former further including an apparatus for applying a plurality of different density layers to at least one density gradient tube (54A-54H); and an apparatus for immobilizing the fluids in said density gradient tube (54A-54H).

**18.** Apparatus in accordance with claim 17 **characterized by** apparatus for mixing said different layers in said density gradient tube (54A-54H) so as to form a continuous gradient in said tube (54A-54H).

**19.** Apparatus in accordance with claim 18 **characterized in that** the apparatus for immobilizing includes an apparatus for freezing.

**20.** The combination of a shipping container and at least one immobilized solution **characterized in that** said immobilized solution is at least part of a gradient density tube (54A-54H).

**21.** The combination of claim 20 **characterized in that** the at one immobilized solution is frozen.

**22.** The combination of claim 20 **characterized in that** the at least one immobilized solution is a sucrose solution.

**25.** The combination of claim 20 **characterized in that** the at least one solution is a plurality of frozen sucrose solutions.

**26.** The combination of claim 20 **characterized in that** the plurality of immobilized solutions are at least one gradient density tube (54A-54H) with at least one gradient density solution in it.
